# EUROPEAN PATENT APPLICATION

(11) **EP 4 060 025 A1**
(43) Date of publication of application: **21.09.2022**
(21) Application number: 20888117.7
(22) Date of filing: 23.10.2020
(51) Int. Cl.: C12N 5/071

(54) **PREPARATION METHOD FOR OLFACTORY PRECURSOR CELL**

(30) Priority: 15.11.2019 CN 201911118936
(71) Applicant: Beijing Hongtianji Neuroscience Academy, Shijingshan District Beijing 100043 (CN)
(72) Inventor: HUANG, Hongyun, Beijing 100043 (CN); GAO, Wenyong, Beijing 100043 (CN); LIU, Ying, Beijing 100043 (CN)
(74) Representative: Potter Clarkson
(86) International application number: PCT/CN2020/123101
(87) International publication number: WO 2021/093550

(57) **Abstract**

Provided is a preparation method for olfactory progenitor cells. Also provided is an olfactory progenitor cell obtained by the method according to the present invention, wherein the single cell of the olfactory progenitor cell can be serially passaged for more than 11 generations. Compared with the prior art, the preparation method for olfactory progenitor cells of the present invention has excellent effects, by which a large quantity of olfactory progenitor cells can be obtained. Moreover, the method is simple and feasible with low cost and good safety, and has a good application prospect in China and abroad.

## Description

### Technical Field

The present invention belongs to the field of cell culture technology, and specifically relates to a preparation method for olfactory progenitor cells.

### Background Art

Olfactory progenitor cells, also referred to as olfactory neural progenitors (ONPs), originate from the olfactory basement membrane and are distributed in the olfactory bulb and olfactory mucosa. Olfactory progenitor cells belong to a class of neural progenitor cells in the ectoderm due to the fact that they can differentiate into olfactory ensheathing cells and olfactory neurons. They have the potential for self-renewal, and directed differentiation and development, can secrete neurotrophic factors and axon growth stimulating substances and promote axonal regeneration and myelin sheath formation. All of these factors can support the extension of axons, and have comprehensive neurorestorative effects including neurotrophy, neuroprotection, immune regulation or stimulation, inhibition of gliosis and scar formation, promotion of myelination and axonal regeneration, and etc. These properties of olfactory progenitor cells can establish a good internal environment for restoration and functional recovery of damaged nerves, making them ideal candidate cells for the treatment of central nervous system diseases and injuries, and have a broad prospect of clinical application.

Therefore, how to culture and expand olfactory progenitor cells safely and effectively *in vitro* has an important influence on their widespread use in basic and clinical application research. However, the research on olfactory progenitor cells in China and abroad in the prior art still has the following defects:
1. when olfactory progenitor cells are prepared, the sampling sites of tissue specimens differentiate greatly, which increase the chance of contamination of both fibroblasts and hematopoietic stem cells;
2. the current preparation methods mostly digest the tissues by an enzymatic process, which limit the quantity, purity and etc. of resultant primary olfactory progenitor cells;
3. the selection of culture medium for the current preparation methods limits the expansion efficiency and cell purity of obtained olfactory progenitor cells;
4. the subculture modes and passage numbers of olfactory progenitor cells in the current preparation methods affect the survival status and purity of obtained olfactory progenitor cells.

In view of the above, there is an urgent need for new preparation methods of olfactory progenitor cells.

### Disclosure of the Invention

With respect to the defects in the prior art, the present invention provides a method for preparing olfactory progenitor cells. The method provided by the present invention can culture and efficiently amplify olfactory progenitor cells *in vitro,* thereby obtaining olfactory progenitor cells with a high purity. Further, the preparation method provided by the present invention optimizes the sampling site of olfactory progenitor cells and method of tissue processing. Compared with the prior art, the method of the present invention breaks through the technical bottleneck involving passage and purity in the current technology, has a great potential in the experimental and clinical application research in China and abroad and a broad application prospect, and provides a powerful guarantee for clinical cell research.

The object of the present invention is achieved through the following technical solutions:
In one aspect, the present invention provides a method for preparing olfactory progenitor cells, the method comprising the following steps of:
(1) taking the olfactory mucosa tissues between the upper turbinate and middle turbinate, and subjecting them to an aseptic treatment;
(2) pretreating the blocks of olfactory mucosa tissue obtained in step (1) with 1-2 times volume of 5% (g/100mL) trypsin aqueous solution and 1-2 times volume of 0.2 mmol/L ethylenediamine tetraacetic acid (EDTA) aqueous solution at 36-38°C for 5-15 minutes;
(3) removing the loose layers of surface and base membranes of the blocks of olfactory mucosa tissue pretreated in step (2), rinsing the blocks of olfactory mucosa tissue with a phosphate buffered solution (PBS) for 2-5 times, and preparing them into small pieces with a size of 0.5-1.5 mm³;
(4) culturing the small pieces processed in step (3) with a olfactory progenitor cell culture medium, and allowing them to grow adherently;
wherein the olfactory progenitor cell culture medium comprises the following components:
basic culture medium DMEM/DF12, epidermal cell growth factor (EGF), fibroblast growth factor (FGF), N2 cell culture supplement, B27 cell culture supplement, bovine serum albumin (BSA), glutamine and NEAA cell culture supplement; and
(5) harvesting single cells when the cells grow adherently to a confluence of 75%-90%, adding the olfactory progenitor cell culture medium to prepare a cell suspension, and obtaining the olfactory progenitor cells;
wherein the above operations are all performed under a strict aseptic condition.

In step (1) of the preparation method according to the present invention, the olfactory mucosa tissues between the upper turbinate and middle turbinate of a donor are taken with a specimen tissue forcep, and the obtained blocks of nasal mucosa tissue are placed into an ice box.

In step (1) of the preparation method according to the present invention, the aseptic processing adopts a medically aseptic operation.

In the preparation method according to the present invention, step (3) is performed under a dissecting microscope.

In the preparation method according to the present invention, step (3) further comprises the following steps of:
preparing the small pieces into little pieces with a size of 0.2-0.4 mm³, and repeatedly rinsing the little pieces with the olfactory progenitor cell culture medium containing 160 U/mL gentamicin;
preferably, step (3) further comprises the following steps of:
   placing the small pieces into the olfactory progenitor cell culture medium containing 160 U/mL gentamicin, rinsing repeatedly, and tearing them into little pieces with a size of 0.2-0.4 mm³ with an ophthalmic tissue forcep; collecting the little pieces into a centrifuge tube, centrifuging for 10-30 minutes at a centrifuge rotational speed of 800-2100 rpm, and discarding the supernatant; re-adding the olfactory progenitor cell culture medium containing 160 U/mL gentamicin, and oscillating the precipitated tissue blocks sufficiently to make them loosen; and centrifuging again for 10-30 minutes at a centrifuge rotational speed of 800-2100 rpm.

In step (4) of the preparation method according to the present invention, the culture device is wetted with a serum stock solution (Gibco 16000044) before the cell culture.

In step (4) of the preparation method according to the present invention, the culture is protected from light and performed under a condition of 37°C and 5% CO₂.

In step (4) of the preparation method according to the present invention, the olfactory progenitor cell culture medium comprises the following components:
90 mL DMEM/DF12 (11320 Gibco), 80 mg EGF (Pepro Tech), 60 mg FGF (Pepro Tech), 2 mL 1% N2 cell culture supplement (Gibco 17502-048) ), 3 mL 2% B27 cell culture supplement (Gibco 17504-044), 150 mg BSA (Sigma), 0.2 mmol/mL glutamine (Gibco), and 2 mL NEAA cell culture supplement (100X, 11140050);
preferably, the olfactory progenitor cell culture medium further comprises the following components:
   a P63 or P53 inhibitor, Pifithrin-α hydrobromide (63208-82-2)/Pifithrin-µ (64984-31-2), with a concentration in the range of 5-20 µmol/L, to inhibit cell growth when the cell production is too fast; or
   a P63 or P53 agonist, PRIMA-1 Met (19980)/Tenovin-1 (13423), with a concentration in the range of 1-10 µmol/L, to promote cell growth when the cell production is too slow.

In steps (4) and step (5) of the preparation method according to the present invention, the olfactory progenitor cell culture medium is changed every three days.

In step (4) of the preparation method according to the present invention, when the cells grow adherently to a confluence of 55%-85%, the small pieces are collected and transferred into a new culture device for repeated culture;
preferably, the small pieces are repeatedly used for 5-7 times to prepare 5-7 batches of olfactory progenitor cells.

In step (5) of the preparation method according to the present invention, harvesting single cells comprising the following steps of:
rinsing the culture device with a DMEM/F12 solution, detaching the adherent single cells with 0.05% (g/100mL) trypsin aqueous solution and 0.2 mmol/L ethylenediamine tetraacetic acid (EDTA) aqueous solution, centrifuging to collect the single cells, and adding the olfactory progenitor cell culture medium to prepare a cell suspension;
preferably, the cell density of the single cell suspension is 1×10⁵/mL-1×10⁶/mL.

In step (5) of the preparation method according to the present invention, the olfactory progenitor cells are passaged every 2-3 days and subcultured for 1-3 generations.

The present invention also provides the olfactory progenitor cells obtained according to the method of the present invention, wherein the single olfactory progenitor cells can be serially passaged for more than 11 generations.

The olfactory progenitor cells provided by the present invention can be prepared as olfactory ensheathing cells or olfactory neuron cells. For details, see Chinese invention patent application Nos. 201510935540.3 or 201510516055.2, the entire disclosures of which are incorporated herein by reference.

Compared with the prior art, the present invention has the following advantages:
1. the method of the present invention optimizes the sampling site of tissue specimens, and combines it with a multi-enzyme pretreatment regimen of the sampled tissues before the culture and removal in the subsequent culture process, leading to the reduction of contamination of hematopoietic stem cells and fibroblasts, and the improvement of cell purity.
2. the method of the present invention can achieve a repeated explant culture and obtain olfactory progenitor cells, thereby saving the source of tissue specimens.
3. by using the method of the present invention, the cell expansion rate, biological activity, purity, and etc. are improved by the olfactory progenitor cell culture medium.
4. by using 0.05% trypsin and 0.2 mmol/L EDTA for enzymatic digestion and passage, the damage to cells is reduced.
5. the olfactory progenitor cells obtained according to the method of the present invention have strong targeting properties and regeneration ability; in addition, the olfactory progenitor cells obtained by the primary culture can be passaged at a ratio of 1: 3 for more than 11 generations, which have still a high purity; the olfactory progenitor cells obtained according to the method of the present invention can be allotransplanted; the present invention can reduce the preparation cost and increase the safety and effectiveness of application research, and thus has a broad of application prospect.

Based on the above, the preparation method for olfactory progenitor cells of the present invention has excellent effects, and can obtain a large quantity of olfactory progenitor cells. Moreover, the method is simple and feasible with low cost and good safety, and has a good application prospect at home and abroad.

### Brief Description of the Drawings

Hereinafter, the embodiments of the present invention will be described in detail with reference to the accompanying drawings, in which:

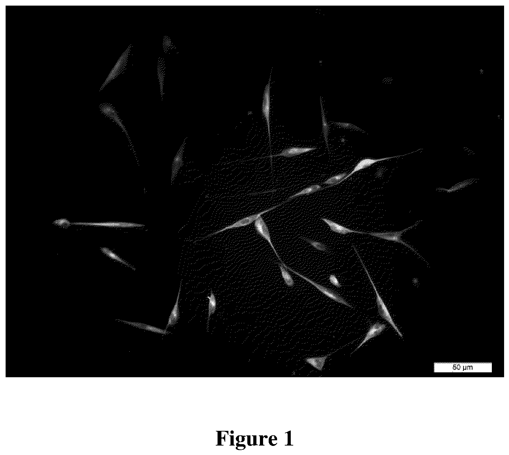

Figure 1 shows the morphology of olfactory progenitor cells prepared according to the method of the present invention.

### Best Modes for Carrying Out the Invention

The present invention will be described in detail below in conjunction with the examples, but is not limited to the following examples. Within the scope of knowledge possessed by those skilled in the art, various changes to the culture medium, cryopreservation protection solution, primary culture time, passage time, multi-batch harvest, and etc. can also be made without departing from the spirit of the present invention.

### Example 1 Preparation of olfactory progenitor cells according to the method of the present invention

An olfactory progenitor cell culture medium was prepared by using 90 mL DMEM/DF12 (11320 Gibco), 80 mg EGF (Pepro Tech) ), 60 mg FGF (Pepro Tech), 2 mL 1% N2 cell culture supplement (Gibco 17502-048), 3 mL 2% B27 cell culture supplement (gibco 17504-044), 150 mg BSA (Sigma), 0.2 mmol/mL glutamine (Gibco) and 2 mL NEAA cell culture supplement (100X, 11140050); and then culture flasks were coated with a serum stock solution (Gibco 16000044) for 24 hours in advance.

Specifically, the method included the following steps of:
(1) obtaining the nasal olfactory mucosa: the blocks of olfactory mucosa tissue between the upper turbinate and middle turbinate of a donor were taken with a specimen tissue forcep, and the obtained blocks of nasal mucosa tissue were placed into a glass dish on an ice box and sent to a culture room immediately for aseptic processing;
(2) the obtained blocks of olfactory mucosa tissue were pretreated with 1 time volume of 5% (g/100mL) trypsin aqueous solution and 1 time volume of 0.2 mmol/L EDTA aqueous solution at 36°C for 15 minutes;
(3) the loose layers of surface and base membranes of the olfactory mucosa tissue blocks were removed under a dissecting microscope, and the olfactory mucosa tissue blocks were rinsed with PBS for 3 times and cut into small pieces with a size of 1 mm³;
   the small pieces were placed into the olfactory progenitor cell culture medium containing 160 U/mL gentamicin, rinsed repeatedly for 3 times, and torn into little pieces with a size of about 0.4 mm³ with an ophthalmic tissue forcep; the tissue blocks were collected into a 10 mL centrifuge tube and centrifuged at a high speed with a centrifuge rotational speed of 1100 rpm for 20 minutes, and the supernatant was discarded; the olfactory progenitor cell culture medium containing 160 U/mL gentamicin was re-added, and the precipitated tissue blocks were oscillated sufficiently to make them loosen and then centrifuged at a high speed again with a centrifuge rotational speed of 1100 rpm for 20 minutes;
(4) the tissue blocks were added into an appropriate amount of culture medium and mixed uniformly, seeded into the pre-coated culture flasks to allow them grow adherently with a distance among the individual tissue blocks being about 4 mm-6 mm, and cultured in an incubator protected from light under 5% CO₂ at 37°C for 3 days; this step was used for the purpose of maximizing the removal of fibroblasts and hematopoietic cells;
(5) the culture flasks were gently shaken to collect the tissue blocks after 3 days of culture, and added with an appropriate amount of the olfactory progenitor cell culture medium; the tissue blocks were seeded into the pre-coated culture flasks with a distance among the individual tissue blocks being about 4 mm-6 mm, and cultured in an incubator protected from light under 5% CO₂ at 37°C;
   the obtained single cells were cultured continuously, and a half of the olfactory progenitor cell culture medium was changed every 3 days; on the 3rd day, the growth of multi-polar cells, such as bipolar and tripolar cells was observed under a microscope;
   the culture flasks were gently shaken to collect the tissue blocks when the single cells grew to a confluence of about 60%; the tissue blocks were seeded into the pre-coated culture flasks with an appropriate distance among the individual tissue blocks being about 4 mm-6 mm, and cultured in an incubator protected from light under 5% CO₂ at 37°C; the adherent single cells in the original cell culture flasks were continued to be cultured by continuously adding the olfactory progenitor cell culture medium;
(6) when the single cells grow to a confluence of 75%-90%, the single cells were harvested by gently rinsing the culture flasks with a DMEM/F12 solution repeatedly for 2 times, detaching the adherent single cells with an aqueous solution containing 0.05% trypsin/0.2 mmol/L EDTA, and centrifuging at a rotational speed of 1100 rpm for 20 minutes; the olfactory progenitor cell culture medium was added to prepare a cell suspension which was divided into 3 parts depending on the cell number, transferred into the pre-coated culture flasks and cultured in an incubator protected from light under 5% CO₂ at 37°C;
   wherein in steps (5) and (6), an appropriate amount of P63 or P53 inhibitor or P63 or P53 agonist was added depending on the observed culture situation; when the cells grew slowly, an appropriate amount of P63 or P53 agonist was added; when the cells grow too fast, an appropriate amount of P63 or P53 inhibitor was added, to make sure that the single cells can be passaged once every 2-3 days and that the primary cells can be isolated from the tissue blocks in 3-5 days;
   steps (5) and (6) were repeated, and the tissue blocks were discarded when they grew and were used repeatedly for 5-7 times; 5-7 batches of primary olfactory progenitor cells can be obtained, and each batch of olfactory progenitor cells were subjected to subculture at a ratio of 1: 3 repeatedly, which allows the cells to expand for 10-11 generations, and can obtain a large quantity of olfactory progenitor cells with a high purity.

### Example 2 Preparation of olfactory progenitor cells according to the method of the present invention

An olfactory progenitor cell culture medium was prepared by using 90 ml DMEM/DF12 (11320 Gibco), 80 mg EGF (Pepro Tech), 60 mg FGF (Pepro Tech), 2 mL 1% N2 cell culture supplement (gibco), 3 mL 2% B27 cell culture supplement (gibco), 150 mg BSA (Sigma), 0.2 mmol/ml glutamine (Gibco), and 2 ml non-essential amino acids (11140050 GIBCO); and then the culture flasks were coated with serum (Gibco 16000044) for 24 hours in advance.

Specifically, the method included the following steps of:
(1) obtaining the nasal olfactory mucosa: the blocks of olfactory mucosa tissue between the upper turbinate and middle turbinate of a donor were taken with a specimen tissue forcep, and the obtained blocks of nasal mucosa tissue were placed into a glass dish on an ice box and sent to a culture room immediately for aseptic processing;
(2) the obtained blocks of olfactory mucosa tissue were treated with 1 time volume of 5% (g/100mL) trypsin and 1 time volume of 0.2 mmol/L EDTA at 38°C for 15 minutes;
(3) the loose layers of surface and base membranes of the olfactory mucosa tissue blocks were removed under a dissecting microscope, and the olfactory mucosa tissue blocks were rinsed with PBS for 3 times and cut into small pieces with a size of 1 mm³;
   the small pieces were placed into the olfactory progenitor cell culture medium containing 160 U/mL gentamicin, rinsed repeatedly for 3 times, and torn into little pieces with a size of about 0.4 mm³ with an ophthalmic tissue forcep; the tissue blocks were collected into a 10 mL centrifuge tube and centrifuged at a high speed with a centrifuge rotational speed of 2100 rpm for 10 minutes, and the supernatant was discarded; the olfactory progenitor cell culture medium containing 160 U/mL gentamicin was re-added, and the precipitated tissue blocks were oscillated sufficiently to make them loosen, and then centrifuged at a high speed again with a centrifuge rotational speed of 2100 rpm for 30 minutes;
(4) the tissue blocks were added into an appropriate amount of culture medium and mixed uniformly, seeded into the pre-coated culture flasks to allow them to grow adherently with an appropriate distance among the individual tissue blocks being about 4 mm-6 mm, and cultured in an incubator protected from light under 5% CO₂ at 37°C for 3 days; this step was used for the purpose of maximizing the removal of fibroblasts and hematopoietic cells;
(5) the culture flasks were gently shaken to collect the tissue blocks after 3 days of culture, and added with an appropriate amount of olfactory progenitor cell culture medium; the tissue blocks were seeded into the pre-coated culture flasks with an appropriate distance among the individual tissue blocks being about 4 mm-6 mm, and cultured in an incubator protected from light under 5% CO₂ at 37°C;
   the obtained single cells were cultured continuously, and a half of olfactory progenitor cell culture medium was changed every 3 days; on the 5th day, the growth of multi-polar cells, such as bipolar and tripolar cells was observed under a microscope;
   the culture flasks were gently shaken to collect the tissue blocks when the single cells grew to a confluence of about 85%; the tissue blocks were seeded into the pre-coated culture flasks with an appropriate distance among the individual tissue blocks being about 4 mm-6 mm, and cultured in an incubator protected from light under 5% CO₂ at 37°C; the adherent single cells in the original cell culture flasks were continued to be cultured by continuously adding the olfactory progenitor cell culture medium;
(6) the single cells were harvested when the cells grew to a confluence of 75%-90% by gently rinsing the culture flasks with a DMEM/F12 solution repeatedly for 2 times, detaching the adherent single cells with an aqueous solution containing 0.05% trypsin/0.2 mmol/L EDTA, and centrifuging at a rotational speed of 2100 rpm for 5 minutes; the olfactory progenitor cell culture medium was added to prepare a cell suspension, which was divided into 3 parts according to the cell number, transferred into the pre-coated culture flasks and cultured in an incubator protected from light under 5% CO₂ at 37°C;
   in steps (5) and (6), an appropriate amount of P63 or P53 inhibitor or P63 or P53 agonist was added according to the observed culture situation; when the cells grew slowly, an appropriate amount of P63 or P53 agonist was added; when the cells grew too fast, an appropriate amount of P63 or P53 inhibitor was added, to make sure that the single cells can be passaged once every 2-3 days and that the primary cells can be isolated from the tissue blocks in 3-5 days.
   steps (5) and (6) were repeated, and the tissue blocks were discarded when they grew and were used repeatedly for 5-7 times; 5-7 batches of primary olfactory progenitor cells can be obtained, and each batch of olfactory progenitor cells were subjected to subculture at a ratio of 1:3 repeatedly, which allows the cells to expand for 10-11 generations, and can obtain a large quantity of olfactory progenitor cells with a high purity.

### Example 3 Identification of olfactory progenitor cells obtained according to the method of the invention

The single cells obtained by culture were identified by conventional immunohistochemical staining, and the results are shown in Figure 1. Cytological identification of olfactory progenitor cells (with nestin, GFAP, and Tuj-1 or OMP cytochemical staining) shows that most cells are nestin positive, while some of them are also GFAP positive and some of them are also Tuj-1 positive, and there are no cells that are only nestin positive, but not positive for other markers for costaining, which are consistent with the characteristics of olfactory progenitor cells.

### Example 4 Preparation of olfactory ensheathing cells or olfactory neuron cells with the olfactory progenitor cells obtained according to the method of the invention

The single cells obtained by culture differentiated directionally to into olfactory ensheathing cells or olfactory neuron cells using the techniques described in Chinese invention patent Nos. 201510935540.3 or 201510516055.2. The cells are used for clinical treatment according to the Clinical Application Guidelines of the International Association of Neurorestoratology.

### Example 5 Application of cells prepared by the present application

Case 1: an American male with a complete thoracic spinal cord (T6) injury for more than nine years had insisted on regular rehabilitation training locally without any recovery of neurological function. He received the treatment with our olfactory ensheathing cells in 2005. After two weeks of treatment, his sensory level decreased and the flexor-extensor strength of hip and knees partially recovered. After returning to the United States, the neurological function continued to improve and recover, and bladder or bowel control partially recovered. Before the treatment, the ASIA score was 50 for motor, 52 for pin prick sensation, and 52 for soft touch. A follow-up after half a year of treatment showed the ASIA score was 56 for motor, 60 for pin prick sensation, and 60 for soft touch sensation. The function index of spinal cord injury according to the International Association of Neurorestoratology was 21 before the treatment and 29 at time of the follow-up after half a year of treatment. The U.S. Fox TV reported that "a patient with an advanced complete spinal cord injury stands up and walks again".

Case 2: an American male was diagnosed with motor neuron disease (amyotrophic lateral sclerosis), and then his condition gradually deteriorated. He staggered with weak strength of upper limbs and hands, and had difficulty in lifting objects. His life expectancy was determined to be only about two years. After receiving our treatment with olfactory ensheathing cell in 2005, the strength of upper and lower limbs and hands increased, and the walk returned to normal. After returning to the United States, he resumed playing golf (he lost the ability to play golf half a year before the treatment with olfactory ensheathing cell), and the therapeutic improvement and function had been maintained for one and a half years. He came back to receive the treatment with olfactory ensheathing cell again when it was difficult for him to play golf again. Then, he received the treatment with olfactory ensheathing cell every one and a half years to two years, and a continued follow-up reported that he was still alive in 2016.

Case 3: an Italian female had suffered from sequelae of cerebral apoplexy in the left side for three years. She can only stand with the aid of a brace on her right ankle, and walk in a claudication gait. She received our treatment with olfactory ensheathing cells in 2006 and then made an gradual improvement of neurological function. A follow-up after two years reported that she did not require a brace for walking, and her gait returned to normal. The Barthel index was 85 before the treatment, and 100 at the time of the follow-up two years later.

The above is a specific description of the present invention in combination with the examples, but the present invention is not limited to the above examples. Within the scope of knowledge possessed by those skilled in the art, various changes can also be made without departing from the spirit of the present invention, and direct or indirect applications of the present invention in other related technical fields are all included in the patent protecting scope of the present invention for the same purpose.

## Claims

1. A preparation method for olfactory progenitor cells, comprising the following steps of:
(1) taking the olfactory mucosa tissues between the upper turbinate and middle turbinate, and subjecting them to an aseptic treatment;
(2) pretreating the olfactory mucosa tissue blocks obtained in step (1) with 1-2 times volume of 5% (g/100mL) trypsin aqueous solution and 1-2 times volume of 0.2 mmol/L EDTA aqueous solution at 36-38°C for 5-15 minutes;
(3) removing the loose layers of surface and base membranes of the olfactory mucosa tissue blocks pretreated in step (2), rinsing the olfactory mucosa tissue blocks with PBS for 2-5 times, and preparing them into small pieces with a size of 0.5-1.5 mm³;
(4) culturing the small pieces treated in step (3) with an olfactory progenitor cell culture medium, and allowing them to grow adherently;
wherein the olfactory progenitor cell culture medium comprises the following components:
basic culture medium DMEM/DF12, epidermal cell growth factor (EGF), fibroblast growth factor (FGF), N2 cell culture supplement, B27 cell culture supplement, bovine serum albumin (BSA), glutamine and NEAA cell culture supplement; and
(5) harvesting single cells when the cells grow adherently to a confluence of 75%-90%, adding the olfactory progenitor cell culture medium to prepare a cell suspension, and obtaining the olfactory progenitor cells;
wherein the above operations are all performed under a strict aseptic condition.

2. The preparation method according to claim 1, wherein in step (1), the olfactory mucosa tissues between the upper turbinate and middle turbinate of a donor are taken with a specimen tissue forcep, and the obtained nasal mucosa tissue blocks are placed into an ice box.

3. The preparation method according to claim 1 or 2, wherein in step (3), the method further comprises the following steps of:
preparing the small pieces into little pieces with a size of 0.2-0.4 mm³, and repeatedly rinsing the little pieces with the olfactory progenitor cell culture medium containing 160 U/mL gentamicin;
preferably, the method comprises the following steps of:
placing the small pieces into the olfactory progenitor cell culture medium containing 160 U/mL gentamicin, rinsing repeatedly, and tearing them into little pieces with a size of 0.2-0.4 mm³ with an ophthalmic tissue forcep; collecting the little pieces into a centrifuge tube and centrifuging at a centrifuge rotational speed of 800-2100 rpm for 10-30 minutes, and discarding the supernatant; re-adding the olfactory progenitor cell culture medium containing 160 U/mL gentamicin, and oscillating the precipitated tissue blocks sufficiently to make them loosen; and centrifuging again at a centrifuge rotational speed of 800-2100 rpm for 10-30 minutes.

4. The preparation method according to any one of claims 1-3, wherein in step (4), the culture device is wetted with a serum stock solution before the cell culture;
preferably, in step (4), the culture are protected from light and performed under a condition of 37°C and 5% CO₂.

5. The preparation method according to any one of claims 1-4, wherein in step (4), the olfactory progenitor cell culture medium comprises the following components:
90 mL DMEM/DF12, 80mg EGF, 60mg FGF, 2mL 1% N2 cell culture supplement, 3 mL 2% B27 cell culture supplement, 150mg BSA, 0.2 mmol/mL glutamine, and 2mL NEAA cell culture supplement;
preferably, the olfactory progenitor cell culture medium further comprises the following components:
5-20 µmol/L P63 or P53 inhibitor, Pifithrin-α hydrobromide or Pifithrin-µ; or
1-10 µmol/L P63 or P53 agonist, PRIMA-1Met/Tenovin-1.

6. The preparation method according to any one of claims 1-5, wherein in steps (4) and step (5), the olfactory progenitor cell culture medium is changed every three days.

7. The preparation method according to any one of claims 1-6, wherein in step (4), when the cells grow adherently to a confluence of 55%-85%, the small pieces are collected and transferred into a new culture device for repeated culture;
preferably, the small pieces are repeatedly used for 5-7 times to prepare 5-7 batches of olfactory progenitor cells.

8. The preparation method according to any one of claims 1-7, wherein in step (5), harvesting the single cells comprising the following steps of:
rinsing the culture device with a DMEM/F12 solution, detaching the adherent single cells with an aqueous solution of 0.05% trypsin and an aqueous solution of 0.2 mmol/L EDTA, centrifuging to collect the single cells, and adding the olfactory progenitor cell culture medium to prepare a cell suspension;
preferably, the cell density of the single cell suspension is 1×10⁵/mL-1×10⁶/mL.

9. The preparation method according to any one of claims 1-8, wherein in step (5), the olfactory progenitor cells are passaged every 2-3 days and subcultured for 1-3 generations.
